# EUROPEAN PATENT SPECIFICATION

(11) **EP 1 697 545 B1**
(45) Date of publication and mention of the grant of the patent: **10.11.2010**
(21) Application number: 04815560.0
(22) Date of filing: 22.12.2004
(51) Int. Cl.: C12Q 1/68, C12P 19/34, C07H 21/02, C07H 21/04

(54) **Multiplex nucleic acid analysis of HPV genotypes**
Multiplexanalyse von Nukleinsäuren zur Bestimmung von Genotypen des humanen Papillomavirus
Analyse multiplex d'acides nucléiques pour déterminer des génotypes du papillomavirus humain

(30) Priority: 23.12.2003 US 532681 P; 26.03.2004 US 556737 P
(43) Date of publication of application: 06.09.2006
(60) Divisional application: 08101207.2
(73) Proprietor: Autogenomics, Inc., Carlsbad, CA 92009 (US)
(72) Inventor: KE, Song-Hua, San Diego, CA (US); HUDSPETH, Richard Loren, San Diego, CA 92131 (US); MAHANT, Vijay K., Murrieta, CA 92562 (US)
(74) Representative: Rupp, Christian
(86) International application number: PCT/US2004/043499
(87) International publication number: WO 2005/064020

(56) References cited:
- WO-A-01/92582
- WO-A-02/103050
- WO-A-03/006677
- US-A1- 2001 053 519
- FAN J-B ET AL: "Parallel genotyping of human SNPs using generic high-density oligonucleotide tag arrays" GENOME RESEARCH, COLD SPRING HARBOR LABORATORY PRESS, WOODBURY, NY, US, vol. 10, no. 6, 2000, pages 853-860, XP002236784 ISSN: 1088-9051
- HIRSCHHORN JOEL N ET AL: "SBE-TAGS: An array-based method for efficient single-nucleotide polymorphism genotyping" PROCEEDINGS OF THE NATIONAL ACADEMY OF SCIENCES OF USA, NATIONAL ACADEMY OF SCIENCE, WASHINGTON, DC, US, vol. 97, no. 22, 24 October 2000 (2000-10-24), pages 12164-12169, XP002158215 ISSN: 0027-8424 -& LINDBLAD-TOH K ET AL: "Large-scale discovery and genotyping of single-nucleotide polymorphisms in the mouse." NATURE GENETICS APR 2000, vol. 24, no. 4, April 2000 (2000-04), pages 381-386, XP002427995 ISSN: 1061-4036
- PASTINEN T ET AL: "A SYSTEM FOR SPECIFIC, HIGH-THROUGPUT GENOTYPING BY ALLELE-SPECIFIC PRIMER EXTENSION ON MICROARRAYS" GENOME RESEARCH, COLD SPRING HARBOR LABORATORY PRESS, WOODBURY, NY, US, vol. 10, no. 7, July 2000 (2000-07), pages 1031-1042, XP008013561 ISSN: 1088-9051
- TAYLOR J D ET AL: "FLOW CYTOMETRIC PLATFORM FOR HIGH-THROUGHPUT SINGLE NUCLEOTIDE POLYMORPHISM ANALYSIS" BIOTECHNIQUES, INFORMA LIFE SCIENCES PUBLISHING, WESTBOROUGH, MA, US, vol. 30, no. 3, March 2001 (2001-03), pages 661-664,666,66, XP001131956 ISSN: 0736-6205
- YE F ET AL: "FLUORESCENT MICROSPHERE-BASED READOUT TECHNOLOGY FOR MULTIPLEXED HUMAN SINGLE NUCLEOTIDE POLYMORPHISM ANALYSIS AND BACTERIAL INDENTIFICATION" HUMAN MUTATION, WILEY-LISS, NEW YORK, NY, US, vol. 17, no. 4, 2001, pages 305-316, XP001118024 ISSN: 1059-7794
- LOVMAR L ET AL: "Microarrays for genotyping human group a rotavirus by multiplex capture and type-specific primer extension" JOURNAL OF CLINICAL MICROBIOLOGY, WASHINGTON, DC, US, vol. 41, no. 11, November 2003 (2003-11), pages 5153-5158, XP002393798 ISSN: 0095-1137
- GHEIT TARIK ET AL: "Development of a sensitive and specific assay combining multiplex PCR and DNA microarray primer extension to detect high-risk mucosal human papillomavirus types." JOURNAL OF CLINICAL MICROBIOLOGY JUN 2006, vol. 44, no. 6, June 2006 (2006-06), pages 2025-2031, XP002427648 ISSN: 0095-1137
- AHERN H.: 'Biochemical Reagent Kits Offer Scientists Good Return on Investment' THE SCIENTIST vol. 9, no. 155, July 1995, pages 20 - 24, XP002939912

### SEQUENCE LISTING

<110> Autogenomics
<120> MULTIPLEXED HPV NUCLEIC ACID ANALYSIS WITH IMPROVED SPECIFICITY
<130> 100788.0023PCT
<150> US 60/532681
   <151> 2003-12-23
<150> US 60/556737
   <151> 2004-03-26
<160> 91
<170> PatentIn version 3.2
<210> 1
   <211> 23
   <212> DNA
   <213> Artificial
<220>
   <223> Artificial sequence; IUPAC ambiguity code applies for all nucleotides other than A, C, G, and T.
<220>
   <221> misc_feature
   <222> (1)..(23)
   <223> Forward Primer for HPV Type 6
<400> 1
   gcaacaacag ttgaagaaga aac 23
<210> 2
   <211> 23
   <212> DNA
   <213> Artificial
<220>
   <223> Artificial sequence; IUPAC ambiguity code applies for all nucleotides other than A, C, G, and T.
<220>
   <221> misc_feature
   <222> (1)..(23)
   <223> Forward Primer for HPV Type 11
<400> 2
   gcacctacag tagaagaaga aac 23
<210> 3
   <211> 20
   <212> DNA
   <213> Artificial
<220>
   <223> Artificial sequence
<220>
   <221> misc_feature
   <222> (1)..(20)
   <223> Forward Primer for HPV Type 16
<220>
   <221> misc_feature
   <222> (12)..(12)
   <223> K is G or T
<220>
   <221> misc_feature
   <222> (15)..(15)
   <223> R is A or G
<400> 3
   gtatatagag akggraatcc 20
<210> 4
   <211> 21
   <212> DNA
   <213> Artificial
   <220>
   <223> Artificial sequence
<220>

   <221> misc_feature
   <222> (1)..(21)
   <223> Forward Primer for HPV Type 18
<400> 4
   gtgtatagag acagtatacc g 21
<210> 5
   <211> 20
   <212> DNA
   <213> Artificial
<220>
   <223> Artificial sequence
<220>
   <221> misc_feature
   <222> (1)..(20)
   <223> Forward Primer for HPV Type 26
<400> 5
   gtatatagag ataggagtcc 20
<210> 6
   <211> 20
   <212> DNA
   <213> Artificial
<220>
   <223> Artificial sequence
<220>
   <221> misc_feature
   <222> (1)..(20)
   <223> Forward Primer for HPV Type 31
<400> 6
   gtatataggg acgacacacc 20
<210> 7
   <211> 20
   <212> DNA
   <213> Artificial
<220>
   <223> Artificial sequence
<220>
   <221> misc_feature
   <222> (1)..(20)
   <223> Forward Primer for HPV Type 33
<220>
   <221> misc_feature
   <222> (12)..(12)
   <223> K is G or T
<220>
   <221> misc_feature
   <222> (15)..(15)
   <223> R is A or G
<400> 7
   gtatatagag akggraatcc 20
<210> 8
   <211> 20
   <212> DNA
   <213> Artificial
<220>
   <223> Artificial sequence
<220>
   <221> misc_feature
   <222> (1)..(20)
   <223> Forward Primer for HPV Type 35
<400> 8
   gtatatagag aaggccagcc 20
<210> 9
   <211> 20
   <212> DNA
   <213> Artificial
<220>
   <223> Artificial sequence
<220>
   <221> misc_feature
   <222> (1) .. (20)
   <223> Forward Primer for HPV Type 39
<400> 9
   gtatataggg acggggaacc 20
<210> 10
   <211> 23
   <212> DNA
   <213> Artificial
<220>
   <223> Artificial sequence
<220>
   <221> misc_feature
   <222> (1)..(23)
   <223> Forward Primer for HPV Type 42
<400> 10
   tggtatacag tggagaaaga aac 23
<210> 11
   <211> 23
   <212> DNA
   <213> Artificial
<220>
   <223> Artificial sequence
<220>
   <221> misc_feature
   <222> (1)..(23)
   <223> Forward Primer for HPV Type 43
<400> 11
   gcagatactg tagaagaaga aac 23
<210> 12
   <211> 23
   <212> DNA
   <213> Artificial
<220>
   <223> Artificial sequence
<220>
   <221> misc_feature
   <222> (1)..(23)
   <223> Forward Primer for HPV Type 44
<400> 12
   gcagtaacag tggaagaaga aac 23
<210> 13
   <211> 20
   <212> DNA
   <213> Artificial
<220>
   <223> Artificial sequence
<220>
   <221> misc_feature
   <222> (1)..(20)
   <223> Forward Primer for HPV Type 45
<400> 13
   gtgtatagag actgtatagc 20
<210> 14
   <211> 26
   <212> DNA
   <213> Artificial
<220>
   <223> Artificial sequence
<220>
   <221> misc_feature
   <222> (1)..(26)
   <223> Forward Primer for HPV Type 51
<400> 14
   gattgtatat agggataata atccat 26
<210> 15
   <211> 25
   <212> DNA
   <213> Artificial
<220>
   <223> Artificial sequence
<220>
   <221> misc_feature
   <222> (1)..(25)
   <223> Forward Primer for HPV Type 52
<400> 15
   gaatagtata tagagacaat aatcc 25
<210> 16
   <211> 20
   <212> DNA
   <213> Artificial
<220>
   <223> Artificial sequence
<220>
   <221> misc_feature
   <222> (1)..(20)
   <223> Forward Primer for HPV Type 53
<400> 16
   gtgtatagag acgggtatcc 20
<210> 17
   <211> 20
   <212> DNA
   <213> Artificial
<220>
   <223> Artificial sequence
<220>
   <221> misc_feature
   <222> (1)..(20)
   <223> Forward Primer for HPV Type 56
<400> 17
   gtgtataggg atgattttcc 20
<210> 18
   <211> 20
   <212> DNA
   <213> Artificial
<220>
   <223> Artificial sequence
<220>
   <221> misc_feature
   <222> (1)..(20)
   <223> Forward Primer for HPV Type 58
<400> 18
   gtgtatagag atggaaatcc 20
<210> 19
   <211> 20
   <212> DNA
   <213> Artificial
<220>
   <223> Artificial sequence
<220>
   <221> misc_feature
   <222> (1)..(20)
   <223> Forward Primer for HPV Type 59
<400> 19
   gtgtatagag actgtagacc 20
<210> 20
   <211> 24
   <212> DNA
   <213> Artificial
<220>
   <223> Artificial sequence
<220>
   <221> misc_feature
   <222> (1)..(24)
   <223> Forward Primer for HPV Type 66
<400> 20
   aactagtata tagaaacaat tggc 24
<210> 21
   <211> 21
   <212> DNA
   <213> Artificial
<220>
   <223> Artificial sequence
<220>
   <221> misc_feature
   <222> (1) .. (21)
   <223> Forward Primer for HPV Type 68
<400> 21
   gtagtatata gggacggggt a 21
<210> 22
   <211> 25
   <212> DNA
   <213> Artificial
<220>
   <223> Artificial sequence
<220>
   <221> misc_feature
   <222> (1)..(25)
   <223> Forward Primer for HPV Type 69
<400> 22
   gaatagtgta tagaaatgat agtgc 25
<210> 23
   <211> 25
   <212> DNA
   <213> Artificial
<220>
   <223> Artificial sequence
<220>
   <221> misc_feature
   <222> (1)..(25)
   <223> Forward Primer for HPV Type 73
<400> 23
   gtattgtata tagaaaggat aaacc 25
<210> 24
   <211> 19
   <212> DNA
   <213> Artificial
<220>
   <223> Artificial sequence
<220>
   <221> misc_feature
   <222> (1)..(19)
   <223> Forward Primer for HPV Type 82
<400> 24
   gtatatagga caatacgcc 19
<210> 25
   <211> 23
   <212> DNA
   <213> Artificial
<220>
   <223> Artificial sequence
<220>
   <221> misc_feature
   <222> (1)..(23)
   <223> Backward Primer for HPV Type 6
<400> 25
   agctgttgca cttctctgat gtc 23
<210> 26
   <211> 23
   <212> DNA
   <213> Artificial
<220>
   <223> Artificial sequence
<220>
   <221> misc_feature
   <222> (1)..(23)
   <223> Backward Primer for HPV Type 11
<400> 26
   aggtcttgta gttgtctgat gtc 23
<210> 27
   <211> 24
   <212> DNA
   <213> Artificial
<220>
   <223> Artificial sequence
<220>
   <221> misc_feature
   <222> (1) .. (24)
   <223> Backward Primer for HPV Type 16
<220>
   <221> misc_feature
   <222> (17)..(17)
   <223> R is A or G
<220>
   <221> misc_feature
   <222> (21)..(21)
   <223> R is A or G
<400> 27
   aattgctcat aacagtrgag rtca 24
<210> 28
   <211> 24
   <212> DNA
   <213> Artificial
<220>
   <223> Artificial sequence
<220>
   <221> misc_feature
   <222> (1)..(24)
   <223> Backward Primer for HPV Type 18
<220>
   <221> misc_feature
   <222> (17)..(17)
   <223> S is C or G
<400> 28
   aattgctcgt gacatasaag gtca 24
<210> 29
   <211> 24
   <212> DNA
   <213> Artificial
<220>
   <223> Artificial sequence
<220>
   <221> misc_feature
   <222> (1)..(24)
   <223> Backward Primer for HPV Type 26
<220>
   <221> misc_feature
   <222> (12)..(12)
   <223> R is A or G
<220>
   <221> misc_feature
   <222> (15)..(15)
   <223> 5 is C or G
<220>
   <221> misc_feature
   <222> (16)..(16)
   <223> Y is C or T
<400> 29
   aattgttcgt arcasygtag gtca 24
<210> 30
   <211> 24
   <212> DNA
   <213> Artificial .
<220>
   <223> Artificial sequence
<220>
   <221> misc_feature
   <222> (1)..(24)
   <223> Backward Primer for HPV Type 31
<220>
   <221> misc_feature
   <222> (17)..(17)
   <223> R is A or G
<220>
   <221> misc_feature
   <222> (21)..(21)
   <223> R is A or G
<400> 30
   aattgctcat aacagtrgag rtca 24
<210> 31
   <211> 24
   <212> DNA
   <213> Artificial
<220>
   <223> Artificial sequence
<220>
   <221> misc_feature
   <222> (1)..(24)
   <223> Backward Primer for HPV Type 33
<220>
   <221> misc_feature
   <222> (12)..(12)
   <223> R is A or G
<400> 31
   aattgctcat arcagtatag gtca 24
<210> 32
   <211> 24
   <212> DNA
   <213> Artificial
<220>
   <223> Artificial sequence
<220>
   <221> misc_feature
   <222> (1)..(24)
   <223> Backward Primer for HPV Type 35
<220>
   <221> misc_feature
   <222> (12)..(12)
   <223> R is A or G
<400> 32
   aattgctcat arcagtatag gtca 24
<210> 33
   <211> 24
   <212> DNA
   <213> Artificial
<220>
   <223> Artificial sequence
<220>
   <221> misc_feature
   <222> (1)..(24)
   <223> Backward Primer for HPV Type 39
<220>
   <221> misc_feature
   <222> (17)..(17)
   <223> S is C or G
<400> 33
   aattgctcgt gacatasaag gtca 24
<210> 34
   <211> 20
   <212> DNA
   <213> Artificial
<220>
   <223> Artificial sequence
<220>
   <221> misc_feature
   <222> (1)..(20)
   <223> Backward Primer for HPV Type 42
<400> 34
   cccaaaagca tctgttgcag 20
<210> 35
   <211> 23
   <212> DNA
   <213> Artificial
<220>
   <223> Artificial sequence
<220>
   <221> misc_feature
   <222> (1)..(23)
   <223> Backward Primer for HPV Type 43
<400> 35
   cagtcttcta gcttcttgat gtc 23
<210> 36
   <211> 23
   <212> DNA
   <213> Artificial
<220>
   <223> Artificial sequence
<220>
   <221> misc_feature
   <222> (1)..(23)
   <223> Backward Primer for HPV Type 44
<400> 36
   agcgtatgta ggtgatggat gtc 23
<210> 37
   <211> 24
   <212> DNA
   <213> Artificial
<220>
   <223> Artificial sequence
<220>
   <221> misc_feature
   <222> (1)..(24)
   <223> Backward Primer for HPV Type 45
<220>
   <221> misc_feature
   <222> (12)..(12)
   <223> R is A or G
<220>
   <221> misc_feature
   <222> (17)..(18)
   <223> M is A or C
<400> 37
   aattgctcgt arcacammag gtca 24
<210> 38
   <211> 24
   <212> DNA
   <213> Artificial
<220>
   <223> Artificial sequence
<220>
   <221> misc_feature
   <222> (1)..(24)
   <223> Backward Primer for HPV Type 51
<220>
   <221> misc_feature
   <222> (9)..(9)
   <223> R is A or G
<220>
   <221> misc_feature
   <222> (11)..(11)
   <223> R is A or G
<220>
   <221> misc_feature
   <222> (20)..(20)
   <223> R is A or G
<400> 38
   aattgctcrt rgcattgcar gtca 24
<210> 39
   <211> 24
   <212> DNA
   <213> Artificial
<220>
   <223> Artificial sequence
<220>
   <221> misc_feature
   <222> (1)..(24)
   <223> Backward Primer for HPV Type 52
<220>
   <221> misc_feature
   <222> (16)..(16)
   <223> W is A or T
<400> 39
   aattgctcat agcagwrtag gtca 24
<210> 40
   <211> 24
   <212> DNA
   <213> Artificial
<220>
   <223> Artificial sequence
<220>
   <221> misc_feature
   <222> (1)..(24)
   <223> Backward Primer for HPV Type 53
<220>
   <221> misc_feature
   <222> (9)..(9)
   <223> R is A or G
<220>
   <221> misc_feature
   <222> (11)..(11)
   <223> R is A or G
<220>
   <221> misc_feature
   <222> (20)..(20)
   <223> R is A or G
<400> 40
   aattgctcrt rgcattgcar gtca 24
<210> 41
   <211> 24
   <212> DNA
   <213> Artificial
<220>
   <223> Artificial sequence
<220>
   <221> misc_feature
   <222> (1)..(24)
   <223> Backward Primer for HPV Type 56
<220>
   <221> misc_feature
   <222> (15)..(15)
   <223> Y is C or T
<400> 41
   aattgctcat tgcaytgtag gtca 24
<210> 42
   <211> 24
   <212> DNA
   <213> Artificial
<220>
   <223> Artificial sequence
<220>
   <221> misc_feature
   <222> (1)..(24)
   <223> Backward Primer for HPV Type 58
<220>
   <221> misc_feature
   <222> (16)..(16)
   <223> W is A or T
<220>
   <221> misc_feature
   <222> (17)..(17)
   <223> R is A or G
<400> 42
   aattgctcat agcagwrtag gtca 24
<210> 43
   <211> 24
   <212> DNA
   <213> Artificial
<220>
   <223> Artificial sequence
<220>
   <221> misc_feature
   <222> (1)..(24)
   <223> Backward Primer for HPV Type 59
<220>
   <221> misc_feature
   <222> (12)..(12)
   <223> R is A or G
<220>
   <221> misc_feature
   <222> (17)..(18)
   <223> M is A or C
<400> 43
   aattgctcgt arcacammag gtca 24
<210> 44
   <211> 24
   <212> DNA
   <213> Artificial
<220>
   <223> Artificial sequence
<220>
   <221> misc_feature
   <222> (1)..(24) for HPV Type 66
   <223> Backward Primer for HPV Type 66
<220>
   <221> misc_feature
   <222> (15)..(15)
   <223> Y is C or T
<400> 44
   aattgctcat tgcaytgtag gtca 24
<210> 45
   <211> 24
   <212> DNA
   <213> Artificial
<220>
   <223> Artificial sequence
<220>
   <221> misc_feature
   <222> (1)..(24)
   <223> Backward Primer for HPV Type 68
<400> 45
   aattgctcat gacatacaag gtcg 24
<210> 46
   <211> 24
   <212> DNA
   <213> Artificial
<220>
   <223> Artificial sequence
<220>
   <221> misc_feature
   <222> (1)..(24)
   <223> Backward Primer for HPV Type 69
<220>
   <221> misc_feature
   <222> (12)..(12)
   <223> R is A or G
<220>
   <221> misc_feature
   <222> (15)..(15)
   <223> 5 is C or G
<220>
   <221> misc_feature
   <222> (16)..(16)
   <223> Y is C or T
<400> 46
   aattgttcgt arcasygtag gtca 24
<210> 47
   <211> 24
   <212> DNA
   <213> Artificial
<220>
   <223> Artificial sequence
<220>
   <221> misc_feature
   <222> (1) .. (24)
   <223> Backward Primer for HPV Type 73
<400> 47
   caatgactcg taacatgtaa ggtc 24
<210> 48
   <211> 24
   <212> DNA
   <213> Artificial
<220>
   <223> Artificial sequence
<220>
   <221> misc_feature
   <222> (1)..(24)
   <223> Backward Primer for HPV Type 82
<220>
   <221> misc_feature
   <222> (9)..(9)
   <223> R is A or G
<220>
   <221> misc_feature
   <222> (11) .. (11)
   <223> R is A or G
<220>
   <221> misc_feature
   <222> (20)..(20)
   <223> R is A or G
<400> 48
   aattgctcrt rgcattgcar gtca 24
<210> 49
   <211> 20
   <212> DNA
   <213> Artificial
<220>
   <223> Artificial sequence
<220>
   <221> misc_feature
   <222> (1) .. (20)
   <223> Extension Primer for HPV Type 6
<400> 49
   gaagtggacg gacaagattc 20
<210> 50
   <211> 20
   <212> DNA
   <213> Artificial
<220>
   <223> Artificial sequence
<220>
   <221> misc_feature
   <222> (1)..(20)
   <223> Extension Primer for HPV Type 11
<400> 50
   caaggtggac aacacagacg 20
<210> 51
   <211> 22
   <212> DNA
   <213> Artificial
<220>
   <223> Artificial sequence
<220>
   <221> misc_feature
   <222> (1)..(22)
   <223> Extension Primer for HPV Type 16
<400> 51
   gcatggagat acacctacat tg 22
<210> 52
   <211> 21
   <212> DNA
   <213> Artificial
<220>
   <223> Artificial sequence
<220>
   <221> misc_feature
   <222> (1)..(21)
   <223> Extension Primer for HPV Type 18
<400> 52
   gacaggaacg actccaacga c 21
<210> 53
   <211> 20
   <212> DNA
   <213> Artificial
<220>
   <223> Artificial sequence
<220>
   <221> misc_feature
   <222> (1)..(20)
   <223> Extension Primer for HPV Type 26
<400> 53
   gagaccaagg cgccaaacag 20
<210> 54
   <211> 22
   <212> DNA
   <213> Artificial
<220>
   <223> Artificial sequence
<220>
   <221> misc_feature
   <222> (1)..(22)
   <223> Extension Primer for HPV Type 31
<400> 54
   catgcgtgga gaaacaccta cg 22
<210> 55
   <211> 21
   <212> DNA
   <213> Artificial
<220>
   <223> Artificial sequence
<220>
   <221> misc_feature
   <222> (1)..(21)
   <223> Extension Primer for HPV Type 33
<400> 55
   cgacgtagag aaactgcact g 21
<210> 56
   <211> 20
   <212> DNA
   <213> Artificial
<220>
   <223> Artificial sequence
<220>
   <221> misc_feature
   <222> (1)..(20)
   <223> Extension Primer for HPV Type 35
<400> 56
   gacaggtcgg tgtatgtcct 20
<210> 57
   <211> 21
   <212> DNA
   <213> Artificial
<220>
   <223> Artificial sequence
<220>
   <221> misc_feature
   <222> (1)..(21)
   <223> Extension Primer for HPV Type 39
<400> 57
   gaccgcagac taacacgaag a 21
<210> 58
   <211> 20
   <212> DNA
   <213> Artificial
<220>
   <223> Artificial sequence
<220>
   <221> misc_feature
   <222> (1)..(20)
   <223> Extension Primer for HPV Type 42
<400> 58
   tgaccaagcc aaacaggaca 20
<210> 59
   <211> 20
   <212> DNA
   <213> Artificial
<220>
   <223> Artificial sequence
<220>
   <221> misc_feature
   <222> (1)..(20)
   <223> Extension Primer for HPV Type 43
<400> 59
   ggaccagcaa gtgaatctac 20
<210> 60
   <211> 20
   <212> DNA
   <213> Artificial
<220>
   <223> Artificial sequence
<220>
   <221> misc_feature
   <222> (1)..(20)
   <223> Extension Primer for HPV Type 44
<400> 60
   cgcaagacgt tacacagcct 20
<210> 61
   <211> 21
   <212> DNA
   <213> Artificial
<220>
   <223> Artificial sequence
<220>
   <221> misc_feature
   <222> (1) .. (21)
   <223> Extension Primer for HPV Type 45
<400> 61
   gaaagacttc gcagacgtag g 21
<210> 62
   <211> 20
   <212> DNA
   <213> Artificial
<220>
   <223> Artificial sequence
<220>
   <221> misc_feature
   <222> (1)..(20)
   <223> Extension Primer for HPV Type 51
<400> 62
   acgtacacga caacgtaacg 20
<210> 63
   <211> 21
   <212> DNA
   <213> Artificial
<220>
   <223> Artificial sequence
<220>
   <221> misc_feature
   <222> (1)..(21)
   <223> Extension Primer for HPV Type 52
<400> 63
   ctgtgaccca agtgtaacgt c 21
<210> 64
   <211> 18
   <212> DNA
   <213> Artificial
<220>
   <223> Artificial sequence
<220>
   <221> misc_feature
   <222> (1)..(18)
   <223> Extension Primer for HPV Type 53
<400> 64
   gaccgggtcg tgcctgac 18
<210> 65
   <211> 21
   <212> DNA
   <213> Artificial
<220>
   <223> Artificial sequence
<220>
   <221> misc_feature
   <222> (1)..(21)
   <223> Extension Primer for HPV Type 56
<400> 65
   gagacaaaca tctagagaac c 21
<210> 66
   <211> 19
   <212> DNA
   <213> Artificial
<220>
   <223> Artificial sequence
<220>
<221> misc_feature
   <222> (1)..(19)
   <223> Extension Primer for HPV Type 58
<400> 66
   gacagggcgc tgtgcagtg 19
<210> 67
   <211> 19
   <212> DNA
   <213> Artificial
<220>
   <223> Artificial sequence
<220>
   <221> misc_feature
   <222> (1)..(19)
   <223> Extension Primer for HPV Type 59
<400> 67
   caaagacaag cgcgtagtg 19
<210> 68
   <211> 24
   <212> DNA
   <213> Artificial
<220>
   <223> Artificial sequence
<220>
   <221> misc_feature
   <222> (1)..(24)
   <223> Extension Primer for HPV Type 66
<400> 68
   gacatacgag tagacaagct acag 24
<210> 69
   <211> 18
   <212> DNA
   <213> Artificial
<220>
   <223> Artificial sequence
<220>
   <221> misc_feature
   <222> (1)..(18)
   <223> Extension Primer for HPV Type 68
<400> 69
   gcagacgcac acggcagg 18
<210> 70
   <211> 20
   <212> DNA
   <213> Artificial
<220>
   <223> Artificial sequence
<220>
   <221> misc_feature
   <222> (1)..(20)
   <223> Extension Primer for HPV Type 69
<400> 70
   ggatgaaaag cgacggttcc 20
<210> 71
   <211> 23
   <212> DNA
   <213> Artificial
<220>
   <223> Artificial sequence
<220>
   <221> misc_feature
   <222> (1)..(23)
   <223> Extension Primer for HPV Type 73
<400> 71
   cggtttcatc aaatagcaga aca 23
<210> 72
   <211> 22
   <212> DNA
   <213> Artificial
<220>
   <223> Artificial sequence
<220>
   <221> misc_feature
   <222> (1)..(22)
   <223> Extension Primer for HPV Type 82
<400> 72
   gtgaaaccca ggtgtaataa cg 22
<210> 73
   <211> 20
   <212> DNA
   <213> Artificial
<220>
   <223> Artificial sequence
<220>
   <221> misc_feature
   <222> (1)..(20)
   <223> Forward primer for HPV Type 16
<400> 73
   gtatatagag atgggaatcc 20
<210> 74
   <211> 20
   <212> DNA
   <213> Artificial
<220>
   <223> Artificial sequence
<220>
   <221> misc_feature
   <222> (1)..(20)
   <223> Forward Primer for HPV Type 33
<400> 74
   gtatatagag agggaaatcc 20
<210> 75
   <211> 24
   <212> DNA
   <213> Artificial
<220>
   <223> Artificial sequence
<220>
   <221> misc_feature
   <222> (1)..(24)
   <223> Backward primer for HPV Type 16
<400> 75
   aattgctcat aacagtagag atca 24
<210> 76
   <211> 24
   <212> DNA
   <213> Artificial
<220>
   <223> Artificial sequence
<220>
   <221> misc_feature
   <222> (1)..(24)
   <223> Backward primer for HPV Type 18
<400> 76
   aattgctcgt gacatagaag gtca 24
<210> 77
   <211> 24
   <212> DNA
   <213> Artificial
<220>
   <223> Artificial sequence
<220>
   <221> misc_feature
   <222> (1)..(24)
   <223> Backward Primer for HPV Type 26
<400> 77
   aattgttcgt agcagcgtag gtca 24
<210> 78
   <211> 24
   <212> DNA
   <213> Artificial
<220>
   <223> Artificial sequence
<220>
   <221> misc_feature
   <222> (1)..(24)
   <223> backward primer for HPV type 31
<400> 78
   aattgctcat aacagtggag gtca 24
<210> 79
   <211> 24
   <212> DNA
   <213> Artificial
<220>
   <223> Artificial sequence
<220>
   <221> misc_feature
   <222> (1)..(24)
   <223> Backward primer for HPV type 33
<400> 79
   aattgctcat agcagtatag gtca 24
<210> 80
   <211> 24
   <212> DNA
   <213> Artificial
<220>
   <223> Artificial sequence
<220>
   <221> misc_feature
   <222> (1) .. (24)
   <223> Backward Primer for HPV Type 35
<400> 80
   aattgctcat aacagtatag gtca 24
<210> 81
   <211> 24
   <212> DNA
   <213> Artificial
<220>
   <223> Artificial sequence
<220>
   <221> misc_feature
   <222> (1)..(24)
   <223> Backward primer for HPV Type 39
<400> 81
   aattgctcgt gacatacaag gtca 24
<210> 82
   <211> 24
   <212> DNA
   <213> Artificial
<220>
   <223> Artificial sequence
<220>
   <221> misc_feature
   <222> (1)..(24)
   <223> Backward Primer for HPV Type 45
<400> 82
   aattgctcgt aacacaacag gtca 24
<210> 83
   <211> 24
   <212> DNA
   <213> Artificial
<220>
   <223> Artificial sequence
<220>
   <221> misc_feature
   <222> (1)..(24)
   <223> Backward primer for HPV type 51
<400> 83
   aattgctcgt agcattgcaa gtca 24
<210> 84
   <211> 24
   <212> DNA
   <213> Artificial
<220>
   <223> Artificial sequence
<220>
   <221> misc_feature
   <222> (1)..(24)
   <223> Backward primer for HPV type 52
<400> 84
   aattgctcat agcagtgtag gtca 24
<210> 85
   <211> 24
   <212> DNA
   <213> Artificial
<220>
   <223> Artificial sequence
<220>
   <221> misc_feature
   <222> (1)..(24)
   <223> Backward primer for HPV type 53
<400> 85
   aattgctcat ggcattgcag gtca 24
<210> 86
   <211> 24
   <212> DNA
   <213> Artificial
<220>
   <223> Artificial sequence
<220>
   <221> misc_feature
   <222> (1)..(24)
   <223> Backward primer for HPV type 56
<400> 86
   aattgctcat tgcactgtag gtca 24
<210> 87
   <211> 24
   <212> DNA
   <213> Artificial
<220>
   <223> Artificial sequence
<220>
   <221> misc_feature
   <222> (1)..(24)
   <223> Backward primer for HPV type 58
<400> 87
   aattgctcat agcagaatag gtca 24
<210> 88
   <211> 24
   <212> DNA
   <213> Artificial
<220>
   <223> Artificial sequence
<220>
   <221> misc_feature
   <222> (1)..(24)
   <223> Backward primer for HPV type 59
<400> 88
   aattgctcgt agcacacaag gtca 24
<210> 89
   <211> 24
   <212> DNA
   <213> Artificial
<220>
   <223> Artificial sequence
<220>
   <221> misc_feature
   <222> (1)..(24)
   <223> Backward primer for HPV type 66
<400> 89
   aattgctcat tgcattgtag gtca 24
<210> 90
   <211> 24
   <212> DNA
   <213> Artificial
<220>
   <223> Artificial sequence
<220>
   <221> misc_feature
   <222> (1)..(24)
   <223> Backward primer for HPV type 69
<400> 90
   aattgttcgt aacactgtag gtca 24
<210> 91
   <211> 24
   <212> DNA
   <213> Artificial
<220>
   <223> Artificial sequence
<220>
   <221> misc_feature
   <222> (1)..(24)
   <223> Backward primer for HPV type 82
<400> 91
   aattgctcgt agcattgcaa gtca 24

## Description

### Field of The Invention

The field of the invention is genetic diagnostics, and especially as it relates to multiplex analysis of a single sample.

### Background of The Invention

Despite recent advances in molecular diagnostics, numerous difficulties still remain. Among other problems, analysis of multiple potential genetic changes in a sample suspected to include a virus or oncogene frequently lead to false positive results, or fail to identify all potential changes as the number of such changes increases. Similar difficulties arise where one or more organisms are subject to genotyping or other genetic analysis.

For example, human papillomavirus (HPV) is now considered a major cause of cervical cancer, killing more than 200,000 women around the world each year. The HPV virus is relatively common and more than 100 distinct types of HPV have been identified, some of which are considered "high-risk" for the development of cancer. Detection of such high-risk types of HPV has significant impact on diagnosis, prevention, treatment and management of cervical cancer in HPV-infected women.

To date, most molecular methods for HPV detection and typing rely on hybridization technologies, including southern blot, dot blot, line blot, and *in situ* hybridization. For example, HybridCapture II from Digene is a nucleic acid hybridization microplate assay based on chemiluminescence for the qualitative detection, and differentiating low-risk from high-risk groups. Other commercially available tests employ similar methods and may detect the presence of various types of HPV in a patient sample. However, known HPV typing methods based on hybridization often lack specificity due to cross-hybridization. Cross-hybridization may result in a false positive signal due to closely related types of HPV (*e.g.,* where a target DNA has only a single or few mismatches to the probes being used). Thus, the accuracy of the test results may be compromised with samples containing multiple viral types with closely related sequences.

To overcome problems associated with cross-hybridization, a number of approaches have been taken. Typically, most of the improvements focus on exact control of the stringency conditions. For example, the specificity of hybridization can be controlled by temperature. However, temperature-specific hybridization may lead to false positive results if probes have a high degree of sequence similarity.

Other efforts included the use of peptide nucleic acids (PNA), a universal base stretch, or modified bases (*e.g.*, super G and C) to alter or otherwise affect hybridization/melting temperature of duplexes. Still further known methods involve use of conformationally locked DNA (*e.g.*, to increase duplex stability), etc. While most of such approaches have provided at least some advantages, various problems nevertheless remain. Among other things, currently known approaches tend to fail to provide a significant difference between the melting and/or hybridization temperature of a perfectly matched hybrid and a single base mismatched hybrid.

Fan, J-B et al., in Genome Research, Cold Spring Harbor Laboratory Press, Woodbury, NY, US, vol. 10, no. 6, 2000, p. 853-860, describes a parallel genotyping of human SNPs using generic high-density oligonucleotide tag arrays. First, marker-specific primers are used in PCR amplifications of genomic regions containing SSNPs. Second, the amplification products are used as templates in single base extension (SBE) reactions using chimeric primers.

Hirschhorn, J. et al., in Proceedings of the National Academy of Sciences of USA, National Academy of Science, Washington D.C.,US, vol. 97, no. 22, 24 October 2000, describes a method for parallel genotyping SNPs by single base extension (SBE) using specific bi-functional primers.

WO 02/103050 A2, describes a detection and typing method for human papillomaviruses based on real-time PCR using self-probing amplicon fluorescent primers. In the detection scheme, the amplicon of a type - or integration status specific HPV nucleic acid is self-probing by virtue of having a non-amplified tag sequence that can hybridize with the same strand of the amplicon.

Therefore, while numerous methods for nucleic acid based testing of HPV and other pathogens are known in the art, all or almost all of them suffer from various problems, which are even more aggravated, where such analysis is performed in a multiplex environment (*e.g.*, a biochip). Consequently, there is still a need to provide improved methods and compositions for molecular diagnostics.

### Summary of the Invention

The present invention is directed to genetic diagnostics in which specificity is substantially improved by using a combination of selected multiplex amplification primers and selected multiplex extension primers, wherein the sequences of the primers are designed to maximize hybridization specificity and extension selectivity in a multiplex reaction.

The present invention relates to a multiplex diagnostic kit as defined in claims 1 and 7. The invention also relates to a synthetic nucleic acid for use as a primer as set out in claim 14. The multiplex diagnostic kit is based on a plurality of amplification primer pairs, and a plurality of extension primers, wherein each of the plurality of amplification primer pairs has a sequence such that (a) a plurality of amplicons produced from a target nucleic acid using the plurality of amplification primer pairs, respectively, includes a sequence difference (mutated position) in a target nucleic acid, (b) the plurality of amplicons is produced in a PCR reaction using the same amplification profile, and wherein each of the plurality of extension primers has a sequence such that (c) each of the plurality of extension primers specifically hybridizes to each of the plurality of amplicons at the same extension temperature, respectively, and selective primer extension for each of the plurality of extension primers is achieved at the same extension profile. Amplification primers and extension primers are those described in SEQ ID: 1 to 72.

Particularly preferred kits further include a biochip to which are coupled in a plurality of distinct positions a plurality of distinct capture probes, respectively, and wherein each of the plurality of capture probes hybridizes with a portion of each of the extension primers, respectively. Most preferably, each of the plurality of the distinct capture probes has a unique sequence distinct from the target nucleic acid. Additionally, contemplated kits can include DNA-dependent DNA polymerase (*e.g.* thermostable, or specifically modified and/or isolated for primer extension), and/or an instruction (*e.g.*, to perform the PCR reaction and primer extension in a single tube).

The multiplex diagnostic kit includes at least two forward amplification primers having a sequence according to SEQ ID NO: 1 to 24, at least two backward amplification primers having a sequence according to SEQ ID NO: 25-48 and at least two extension primers having a sequence according to SEQ ID NO: 49-72. Such kits can further include an instruction to perform a multiplex PCR using the at least two forward amplification primers and the at least two backward amplification primers using the same amplification profile, and optionally an instruction to perform a primer extension reaction using the at least two extension primers at the same extension profile (typically in a single test tube).

Additionally, or alternatively, a biochip is included in the test kit to which are coupled in a plurality of distinct positions a plurality of distinct capture probes, respectively, and wherein each of the plurality of capture probes hybridizes with a portion of each of the extension primers, respectively. Most preferably, each of the plurality of the distinct capture probes has a unique sequence distinct from a target nucleic acid to which the amplification primers bind. Where desired, contemplated kits may also include a reagent and/or an enzyme.

In an other contemplated aspect of the inventive subject matter, a synthetic nucleic acid has less than forty nucleotides and comprises an HPV recognition sequence selected from the group consisting of SEQ ID NOs: 1 to 3 and 5 to 24 wherein no more than two nucleotides in the HPV recognition sequence are replaced by A, G, C, or T.

Various objects, features, aspects and advantages of the present invention will become more apparent from the following detailed description of preferred embodiments of the invention.

### Detailed Description

The inventors have unexpectedly discovered that a plurality of potential variants of a single gene can be identified in a single sample using a multiplex test in which amplification primers are used to specifically amplify a target sequence in that gene, wherein the amplicon includes at least one of the potential variants, and wherein extension primers are used to form an extension product that is specific to a variant of the gene.

It should be especially noted that the specificity in such tests is substantially increased over conventional methods by the manner of primer selection. Specifically, the amplification primers are selected to have a sequence such that (a) a plurality of amplicons produced from a target nucleic acid using the amplification primers include sequence difference in a target nucleic acid, and (b) the plurality of amplicons is produced in a PCR reaction using the same amplification profile. In the same test, the extension primers are selected to have a sequence such that (c) the extension primers specifically hybridize to the corresponding amplicons at the same extension temperature (preferably such that the 3'-end of each of the extension primers corresponds to a complementary position of the mutated position), and (d) selective primer extension for each of the extension primers is achieved at the same extension temperature.

Most preferably, the 5'-end of the extension primers further includes a tag (zipcode sequence) that is substantially not (typically less than 70%, and most typically less than 50%) complementary to the sequence of the amplicons and/or the sequence of the target gene, wherein the zipcode sequence is employed to hybridize with a capture probe (preferably on a biochip in a predetermined position). While not limiting to the inventive subject matter, the zipcode has typically a length between about two and twenty, more preferably between five and fifteen, and most preferably between eight and twelve nucleotides, wherein the tags of each of the extension primers are distinct (*i.e.*, have a unique sequence), and wherein the zipcodes (and with that the distinct capture probes) have a unique sequence distinct from the target nucleic acid.

With respect to the particular sequences of the amplification primers and the extension primers, it should be recognized that all sequences are deemed suitable and that the specific sequences will predominantly depend on the particular nature of the target nucleic acid and type of sequence difference that is to be detected. For example, suitable target nucleic acids include native and recombinant DNA (*e.g.,* linear, circular, etc.), RNA (*e.g.,* snRNA, hnRNA, mRNA, etc.), synthetic nucleic acids (*e.g.*, phosphorothioates, PNA, etc.), all of which may be present in, or isolated from a biological source (*e.g.*, biopsy, cell culture, swab, filtrate, plant material, etc.), a non-biological source (*e.g.*, food, soil, water, oil, etc.), or may be entirely synthetic (*e.g.*, on solid phase). Thus, it should be recognized that the length of contemplated target nucleic acids may vary considerably, and is typically between about 50 nucleotides to the length of an entire genome, chromosome, vector, chromosomal fragment, or transcript. Most preferably, the target nucleic acid is a viral or bacterial genome, or a nucleic acid comprising an oncogene, tumor suppressor gene, or other gene that is associated with a predisposition or presence of a disease. In the example, below, a particularly preferred target DNA is a viral DNA, and especially HPV DNA.

It is generally preferred that the amplification primers have a length of between about 12 to 50 nucleotides, and more preferably between about 16 to 30 nucleotides, wherein the amplification primers may additionally (or optionally) include one or more nucleotides that provide one or more desirable properties. For example, contemplated amplification primers may include one or more nucleotides that render the primer (and/or amplicon) quantifiable and typical examples include radiolabeled nucleotides, fluorescence-labeled nucleotides, etc. In another example, contemplated amplification primers may also include one or more nucleotides that will facilitate specific isolation of the primer and/or amplicon (*e.g.*, biotinylated nucleotide). Thus, amplicons generated by contemplated methods may be quantified to normalize a test result, especially where the test result provides a quantitative measure. Amplicons generated by contemplated tests will typically have a length of between about 50 to several thousand nucleotides.

Similarly, preferred extension primers can have a length of between about 12 to 50 nucleotides, and more preferably between about 16 to 30 nucleotides, wherein the extension primers may additionally (or optionally) include one or more nucleotides that provide for one or more desirable properties. For example, particularly contemplated extension primers can include several additional nucleotides that allow specific hybridization of the additional nucleotides to a capture probe. Most preferably, the additional nucleotides have a sequence that is distinct from the sequence of the target nucleic acid (and even more typically of the amplicon). Therefore, capture of the extension product is independent of the target sequence, which further increases selectivity of the test. For example, various SNP-specific tests known in the art use solid-phase or otherwise immobilized extension primers, which tend to produce false positive results where the sequence difference among various mutant sequences allows cross-hybridization. Of course, it should be recognized that the sequence of the extension primer is selected from a sequence available in the amplicon.

Depending on the particular target nucleic acid, contemplated amplification and/or extension primers can also include modified nucleotides and/or have one or more ambiguous positions (*i.e.*, a position in which different nucleotides are present among otherwise identical primers). Ambiguous positions are denoted using the IUPAC nomenclature (R is A or G, Y is C or T, S is C or G, W is A or T, K is G or T, M is A or C, B is C or G or T, D is A or G or T, H is A or C or T, V is A or C or G, and N is A or C or G or T). Therefore, contemplated amplification and/or extension primers may have a single defined Tₘ at a particular solvent and temperature, or several distinct Tₘ.

However, it should be recognized that the amplification primers are chosen such that a multiplex PCR using the amplification primers can be performed using a single amplification profile (wherein the term "amplification profile" refers to a specific combination of denature temperature and time, anneal temperature and time, and polymerization temperature and time), and that all amplicons produced from the multiplex PCR can be used for a primer extension reaction using the extension primers at a single extension temperature (wherein the term "extension temperature" refers to a specific combination of hybridization temperature and time and polymerization temperature and time). Preferred extension products are typically in the range of about 50 to several thousand bases, and it is especially preferred that the extension product includes one or more detectable (and more preferably quantifiable) label. For example, the extension reaction may be performed using one or more directly or indirectly labeled nucleotides, including nucleotides that carry a fluorescent, luminescent, or radioactive label (wherein the molar fraction of labeled nucleotide may be adjusted as appropriate), and/or nucleotides that carry an affinity marker (*e.g.*, biotin, digitoxin) that binds a labeled compound or compound that can otherwise be detected and/or quantified.

It is generally preferred that the extension primer has a sequence and is positioned such that proper hybridization of the extension primer (and especially correct hybridization of the terminal three 3' bases, more preferably terminal two 3' bases, and most preferably terminal 3' base) with the target nucleic acid will result in a detectable extension event. Typically the detectable event is a DNA polymerase-dependent DNA synthesis, wherein at least one of the nucleotides is labeled. Alternatively, the detectable event may also be a DNA ligation using a labeled fragment that abuts with it's 5'-end the 3'-end of the extension primer. With respect to the type of sequence difference that can be detected using contemplated methods, it should be recognized that all known sequence differences are suitable so long as information is available that allows design of the amplification primers and the extension primers. Thus, contemplated differences include deletions, insertions, translocations, and substitutions (*e.g.*, transversion or transition). Furthermore, it should be noted that contemplated sequence differences also include sequence differences found in distinct viral genotypes. Thus, the nucleotide differences between or among various genotypes of a viral species are also considered mutations herein.

Detection is preferably carried out on a biochip or other carrier onto which are immobilized in predetermined positions a plurality of capture probes that hybridize with at least a portion of the extension primer and/or extension product. Therefore, contemplated diagnostic kits can also include (next to contemplated amplification primers and/or extension primers) a biochip to which are coupled in a plurality of distinct positions a plurality of distinct capture probes, respectively, and wherein each of the plurality of capture probes hybridizes with at least a portion of each of the extension primers, respectively. In still further preferred aspects, contemplated capture probes may also include a fluorescent label, wherein the emission of the label is most preferably at a wavelength different from the detection wavelength of the extension product (*e.g.*, Cy5 for the capture probe and Cy3 for the extension product). Among other advantages, such configurations allow normalization and/or calibration of a signal from the extension product. Additionally, or alternatively, suitable kits may include various enzymes (*e.g.*, DNA-dependent DNA polymerase, ligase, etc.), buffers, and other reagents (*e.g.*, labeled and unlabeled nucleotides).

**Table 1A** shows the forward and backward amplification primers and corresponding extension primers for detection of genetic variants of HPV, wherein an extension primer in the same row as a forward and backward amplification primer will bind to the amplicon produced by the amplification primers. It should be noted that the primers in the Table 1A may include degenerate nucleotide positions. Therefore, primers with degenerate positions represent both individual sequences as well as mixtures of sequences defined by the ambiguity codes (*e.g.,* ASA may represent AGA individually or ACA individually, but also a mixture of ACA and AGA together). **Table 1B** depicts a selection of certain primers of Table 1A with non-degenerate sequences.

**TABLE 1A**

| **HPV** | **SEQ ID.** | **Upstream Primer** | **SEQ ID.** | **Downstream Primer** | **SEQ ID.** | **Extension Primer** |
|---|---|---|---|---|---|---|
| 6 | A1 | GCAACAACAGTTGAAGAAGAAAC | B1 | AGCTGTTGCACTTCTCTGATGTC | C1 | GAAGTGGACGGACAAGATTC |
| 11 | A2 | GCACCTACAGTAGAAGAAGAAAC | B2 | AGGTCTTGTAGTTGTCTGATGTC | C2 | CAAGGTGGACAACACAGACG |
| 16 | A3 | GTATATAGAGAKGGRAATCC | B3 | AATTGCTCATAACAGTRGAGRTCA | C3 | GCATGGAGATACACCTACATTG |
| 18 | A4 | GTGTATAGAGACAGTATACCG | B4 | AATTGCTCGTGACATASAAGGTCA | C4 | GACAGGAACGACTCCAACGAC |
| 26 | A5 | GTATATAGAGATAGGAGTCC | B5 | AATTGTTCGTARCASYGTAGGTCA | C5 | GAGACCAAGGCGCCAAACAG |
| 31 | A6 | GTATATAGGGACGACACACC | B6 | AATTGCTCATAACAGTRGAGRTCA | C6 | CATGCGTGGAGAAACACCTACG |
| 33 | A7 | GTATATAGAGAKGGRAATCC | B7 | AATTGCTCATARCAGTATAGGTCA | C7 | CGACGTAGAGAAACTGCACTG |
| 35 | A8 | GTATATAGAGAAGGCCAGCC | B8 | AATTGCTCATARCAGTATAGGTCA | C8 | GACAGGTCGGTGTATGTCCT |
| 39 | A9 | GTATATAGGGACGGGGAACC | B9 | AATTGCTCGTGACATASAAGGTCA | C9 | GACCGCAGACTAACACGAAGA |
| 42 | A10 | TGGTATACAGTGGAGAAAGAAAC | B10 | CCCAAAAGCATCTGTTGCAG | C10 | TGACCAAGCCAAACAGGACA |
| 43 | A11 | GCAGATACTGTAGAAGAAGAAAC | B11 | CAGTCTTCTAGCTTCTTGATGTC | C11 | GGACCAGCAAGTGAATCTAC |
| 44 | A12 | GCAGTAACAGTGGAAGAAGAAAC | B12 | AGCGTATGTAGGTGATGGATGTC | C12 | CGCAAGACGTTACACAGCCT |
| 45 | A13 | GTGTATAGAGACTGTATAGC | B13 | AATTGCTCGTARCACAMMAGGTCA | C13 | GAAAGACTTCGCAGACGTAGG |
| 51 | A14 | GATTGTATATAGGGATAATAATCCAT | B14 | AATTGCTCRTRGCATTGCARGTCA | C14 | ACGTACACGACAACGTAACG |
| 52 | A15 | GAATAGTATATAGAGACAATAATCC | B15 | AATTGCTCATAGCAGWRTAGGTCA | C15 | CTGTGACCCAAGTGTAACGTC |
| 53 | A16 | GTGTATAGAGACGGGTATCC | B16 | AATTGCTCRTRGCATTGCARGTCA | C16 | GACCGGGTCGTGCCTGAC |
| 56 | A17 | GTGTATAGGGATGATTTTCC | B17 | AATTGCTCATTGCAYTGTAGGTCA | C17 | GAGACAAACATCTAGAGAACC |
| 58 | A18 | GTGTATAGAGATGGAAATCC | B18 | AATTGCTCATAGCAGWRTAGGTCA | C18 | GACAGGGCGCTGTGCAGTG |
| 59 | A19 | GTGTATAGAGACTGTAGACC | B19 | AATTGCTCGTARCACAM MAGGTCA | C19 | CAAAGACAAGCGCGTAGTG |
| 66 | A20 | AACTAGTATATAGAAACAATTGGC | B20 | AATTGCTCATTGCAYTGTAGGTCA | C20 | GACATACGAGTAGACAAGCTACAG |
| 68 | A21 | GTAGTATATAGGGACGGGGTA | B21 | AATTGCTCGTGACATACAAGGTCG | C21 | GCAGACGCACACGGCAGG |
| 69 | A22 | GAATAGTGTATAGAAATGATAGTGC | B22 | AATTGTTCGTARCASYGTAGGTCA | C22 | GGATGAAAAGCGACGGTTCC |
| 73 | A23 | GTATTGTATATAGAAAGGATAAACC | B23 | CAATGACTCGTAACATGTAAGGTC | C23 | CGGTTTCATCAAATAGCAGAACA |
| 82 | A24 | GTATATAGGACAATACGCC | B24 | AATTGCTCRTRGCATTGCARGTCA | C24 | GTGAAACCCAGGTGTAATAACG |

**TABLE 1B**

| **HPV** | **SEQ ID.** | **Upstream Primer** | **SEQ ID.** | **Downstream Primer** | **SEQ ID.** | **Extension Primer** |
|---|---|---|---|---|---|---|
| 16 | A25 | GTATATAGAGATGGGAATCC | B25 | AATTGCTCATAACAGTAGAGATCA | C3 | GCATGGAGATACACCTACATTG |
| 18 | A4 | GTGTATAGAGACAGTATACCG | B26 | AATTGCTCGTGACATAGAAGGTCA | C4 | GACAGGAACGACTCCAACGAC |
| 26 | A5 | GTATATAGAGATAGGAGTCC | B27 | AATTGTTCGTAGCAGCGTAGGTCA | C5 | GAGACCAAGGCGCCAAACAG |
| 31 | A6 | GTATATAGGGACGACACACC | B28 | AATTGCTCATAACAGTGGAGGTCA | C6 | CATGCGTGGAGAAACACCTACG |
| 33 | A26 | GTATATAGAGAGGGAAATCC | B29 | AATTGCTCATAGCAGTATAGGTCA | C7 | CGACGTAGAGAAACTGCACTG |
| 35 | A8 | GTATATAGAGAAGGCCAGCC | B30 | AATTGCTCATAACAGTATAGGTCA | C8 | GACAGGTCGGTGTATGTCCT |
| 39 | A9 | GTATATAGGGACGGGGAACC | B31 | AATTGCTCGTGACATACAAGGTCA | C9 | GACCGCAGACTAACACGAAGA |
| 45 | A13 | GTGTATAGAGACTGTATAGC | B32 | AATTGCTCGTAACACAACAGGTCA | C13 | GAAAGACTTCGCAGACGTAGG |
| 51 | A14 | GATTGTATATAGGGATAATAATCCAT | B33 | AATTGCTCGTAGCATTGCAAGTCA | C14 | ACGTACACGACAACGTAACG |
| 52 | A15 | GAATAGTATATAGAGACAATAATCC | B34 | AATTGCTCATAGCAGTGTAGGTCA | C15 | CTGTGACCCAAGTGTAACGTC |
| 53 | A16 | GTGTATAGAGACGGGTATCC | B35 | AATTGCTCATGGCATTGCAGGTCA | C16 | GACCGGGTCGTGCCTGAC |
| 56 | A17 | GTGTATAGGGATGATTTTCC | B36 | AATTGCTCATTGCACTGTAGGTCA | C17 | GAGACAAACATCTAGAGAACC |
| 58 | A18 | GTGTATAGAGATGGAAATCC | B37 | AATTGCTCATAGCAGAATAGGTCA | C18 | GACAGGGCGCTGTGCAGTG |
| 59 | A19 | GTGTATAGAGACTGTAGACC | B38 | AATTGCTCGTAGCACACAAGGTCA | C19 | CAAAGACAAGCGCGTAGTG |
| 66 | A20 | AACTAGTATATAGAAACAATTGGC | B39 | AATTGCTCATTGCATTGTAGGTCA | C20 | GACATACGAGTAGACAAGCTACAG |
| 69 | A22 | GAATAGTGTATAGAAATGATAGTGC | B40 | AATTGTTCGTAACACTGTAGGTCA | C22 | GGATGAAAAGCGACGGTTCC |
| 82 | A24 | GTATATAGGACAATACGCC | B41 | AATTGCTCGTAGCATTGCAAGTCA | C24 | GTGAAACCCAGGTGTAATAACG |

The amplification primers and extension primers correspond to the sequences provided in the sequence listing below, wherein SEQ ID: A1-A24 of Table 1A correspond to Sequence Numbers 1-24 of the sequence listing, respectively, wherein SEQ ID: B1-B24 of Table 1 correspond to Sequence Numbers 25-48 of the sequence listing, respectively, and wherein SEQ ID: C1-C24 of Table 1 correspond to Sequence Numbers 49-72 of the sequence listing, respectively. Furthermore, SEQ ID: A25-A26 of Table 1B correspond to Sequence Numbers 73-74 of the sequence listing, respectively, and SEQ ID: B25-B41 of Table 1B correspond to Sequence Numbers 75-91 of the sequence listing, respectively.

The primers according to Tables 1A and 1B can further be modified to yield a synthetic nucleic acid having less than forty nucleotides and comprising an HPV recognition sequence selected from the group consisting of SEQ ID NOs: 1 to 3 and 5 to 24 wherein no more than two nucleotides in the HPV recognition sequence are replaced by A, G, C or T or other non-natural nucleotide.

Especially contemplated kits will include at least two, more typically at least three to five, and most typically at least ten to twenty of the amplification primer pairs, and/or corresponding extension primers. In such kits, the PCR reaction and/or the primer extension is preferably performed in a single tube, which may be reflected in an instruction accompanying such kits. Alternatively, at least one of the PCR reaction and the primer extension can also be performed in an automated analyzer. Contemplated instructions may further provide information to perform the multiplex PCR using at least two forward amplification primers and at least two backward amplification primers using the same amplification profile, and/or information to perform the primer extension reaction using at least two extension primers at the same extension temperature.

While not wishing to be bound by a particular hypothesis or theory, the inventors contemplate that the specificity of the tests according to the inventive subject matter is further improved by virtue of the fact that at least one of the hybridizations, and more preferably both hybridizations (*i.e.*, for amplification and extension) is performed in solution rather than on a solid phase (which is thought to interfere with hybridization specificity). Furthermore, by the particular choice of primer selection, and especially by targeting distinguishing sequences among a plurality of otherwise similar or identical sequences, hybridization specificity of the amplification and/or extension primers is further increased.

### Experiments

The following experiments were performed to provide exemplary guidance for a test to detect and genotype an HPV virus from a human sample. Here, HPV DNA was isolated from a pap smear using the Qiagen DNA isolation kit and an aliquot of the eluent was subjected to an off-line multiplex PCR using the forward and backward amplification primers with the SEQ ID A1-A24 and B1-B24, respectively. The PCR conditions were as follows:

### HPV Multiplex PCR

To 1 uL sample were added 18.75 uL HPV Amplification Solution, 0.25 uL (1.25 units) Platinum Taq Polymerase (Invitrogen) to a final volume of 20 uL. The HPV Amplification Solution was 21.34 mM Tris-HCL (pH 8.4), 53.35 mM KCL, 2.67 mM MgCl2, 33.34 uM dATP, 33.34 uM dGTP, 33.34 uM dTTP, 6.67 uM dCTP, and 26.68 to 80.03 nM for each of the forward and backward amplification primers.

A 24-plex PCR using the primers of Table 1A was performed using the following amplification profile: Activation of Platinum Taq polymerase was performed by incubation at 94°C for 1 min followed by 40 cycles of 5 sec denaturation at 94°C, 30 sec annealing at 52°C and 40 sec elongation at 72°C. The contents of the multiplex PCR was then used in a subsequent primer extension using the extension oligos with the SEQ ID C1-C24 in a single well of a multi-well plate that was disposed in an automated analyzer as follows:

### HPV Primer Extension

The primer extension was performed in a 24 well plate in an automated analyzer using temperature controlled incubation of the extension mixture and reagents as follows:
To the 20 uL volume from the multiplex PCR reaction were added 20 uL HPV Primer Extension Solution to a final volume of 40 uL. The HPV Primer Extension Solution was 20 mM Tris-HCL (pH 8.4), 50 mM KCL, 2.5 mM MgCl2, 31.25 uM dATP, 31.25 uM dGTP, 31.25 uM dTTP, 5 uM cy5dCTP, and 25 nM for each of the extension primers SEQ ID C1-C24. The extension profile was as follows: The PCR reaction was denatured at 94°C for 1 min followed by 40 cycles of 5 sec at 94°C and 10 sec at 51°C.

### HPV Detection and Genotyping

The entire volume of the extension reaction was then transferred onto a biochip that included in predetermined position a plurality of capture nucleotides as described in our copending International applications WO 03/050591 and WO 02/057416, both of which are incorporated by reference herein. Extension products were detected by fluorescence detection using the Cy label on the extension product (which is only formed where the extension primer forms a perfect hybrid with the target nucleic acid), wherein a predetermined position of the capture primer corresponds to a predetermined HPV genotype. Genotyping and detection was confirmed using the reference test HC2 HPV DNA test from Digene Corporation. The results from the test according to the inventive subject matter and the commercially available test correlated 100% as shown in Table 2 in which HR represents "high-risk" genotype (with particular genotype provided in parentheses), LR represents "low-risk" genotype (with particular genotype provided in parentheses), and neg represents negative result

**Table 2**

| **INVENTIVE TEST (24 PLEX)** | **REFERENCE TEST** |
|---|---|
| HR (45) | HR |
| neg | neg |
| neg | neg |
| HR (31, 66) | HR, LR |
| HR (16, 26, 33, 52, 82), LR (6) | HR, LR |
| HR (16) | HR, LR |
| HR (35, 58, 66) | HR, LR |
| HR (18, 53, 56), LR (42) | HR, LR |
| HR (39, 58) | HR |
| HR (35) | HR |
| neg | neg |
| HR (35, 52) | HR |
| HR (18) | HR |
| HR (18, 35) | HR, LR |

## Claims

1. A multiplex diagnostic kit that allows concurrent detection of a plurality of HPV genotypes selected from 6, 11, 16, 18, 26, 31, 33, 35, 39, 42, 43, 44, 45, 51, 52, 53, 56, 58, 59, 66, 68, 69, 73 and 82 comprising: a plurality of amplification primer pairs and a plurality of extension primers, wherein each of the plurality of amplification primer pairs has a sequence such that
(a) a plurality of amplicons produced from a target nucleic acid using the plurality of amplification primer pairs, respectively, includes a mutated position in a target nucleic acid;
(b) the plurality of amplicons is produced in a PCR reaction using the same amplification profile; and wherein each of the plurality of extension primers has a sequence such that
(c) each of the plurality of extension primers specifically hybridizes to each of the plurality of amplicons at the same extension temperature, respectively, such that the 3'-end of each of the extension primers corresponds to a complementary position of the mutated position, respectively; and
(d) selective primer extension for each of the plurality of extension primers is achieved at the same extension temperature;
which includes at least two amplification primer pairs including forward and backward amplification primers and at least two corresponding extension primers having the following sequences:
SEQ ID:1, SEQ ID:25 and SEQ ID:49;
SEQ ID:2, SEQ ID:26 and SEQ ID:50;
SEQ ID:3, SEQ ID:27 and SEQ ID:51;
SEQ ID:4, SEQ ID:28 and SEQ ID:52;
SEQ ID:5, SEQ ID:29 and SEQ ID:53;
SEQ ID:6, SEQ ID:30 and SEQ ID:54;
SEQ ID:7, SEQ ID:31 and SEQ ID:55;
SEQ ID:8, SEQ ID:32 and SEQ ID:56;
SEQ ID:9, SEQ ID:33 and SEQ ID:57;
SEQ ID:10, SEQ ID:34 and SEQ ID:58;
SEQ ID:11, SEQ ID:35 and SEQ ID:59;
SEQ ID:12, SEQ ID:36 and SEQ ID:60;
SEQ ID:13, SEQ ID:37 and SEQ ID:61;
SEQ ID:14, SEQ ID:38 and SEQ ID:62;
SEQ ID:15, SEQ ID:39 and SEQ ID:63;
SEQ ID:16, SEQ ID:40 and SEQ ID:64;
SEQ ID:17, SEQ ID:41 and SEQ ID:65;
SEQ ID:18, SEQ ID:42 and SEQ ID:66;
SEQ ID:19, SEQ ID:43 and SEQ ID:67;
SEQ ID:20, SEQ ID:44 and SEQ ID:68;
SEQ ID:21, SEQ ID:45 and SEQ ID:69;
SEQ ID:22, SEQ ID:46 and SEQ ID:70;
SEQ ID:23, SEQ ID:47 and SEQ ID:71 and
SEQ ID:24, SEQ ID:48 and SEQ ID:72
whereby the primers with degenerate positions may be represented by the individual sequences or by a mixture of the sequences.

2. The multiplex diagnostic kit of claim 1 further comprising a biochip to which are coupled in a plurality of distinct positions a plurality of distinct capture probes, respectively, and wherein each of the plurality of capture probes hybridizes with a portion of each of the extension primers, respectively.

3. The multiplex diagnostic kit of claim 2 wherein each of the plurality of the distinct capture probes has a unique sequence distinct from the target nucleic acid.

4. The multiplex diagnostic kit of claim 1 further comprising a DNA-dependent DNA polymerase.

5. The multiplex diagnostic kit of claim 1 wherein each of the extension primers further comprises a unique tag at the 5'-end of the extension primers to allow hybridization with a unique capture probe.

6. The multiplex diagnostic kit of claim 1 further comprising an instruction to perform the PCR reaction and primer extension in a single tube.

7. A multiplex diagnostic kit for concurrent detection of a plurality of HPV genotypes selected from 16, 18, 26, 31, 33, 35, 39, 45, 51, 52, 53, 56, 58, 59, 66, 69 and 82, comprising at least two forward amplification primers and at least two backward amplification primers and corresponding extension primers having the following sequences:
SEQ ID:73, SEQ ID:75 and SEQ ID:51;
SEQ ID:4, SEQ ID:76 and SEQ ID:52;
SEQ ID:5, SEQ ID:77 and SEQ ID:53;
SEQ ID:6, SEQ ID:78 and SEQ ID:54;
SEQ ID:74, SEQ ID:79 and SEQ ID:55;
SEQ ID:8, SEQ ID:80 and SEQ ID:56;
SEQ ID:9, SEQ ID:81 and SEQ ID:57;
SEQ ID:13, SEQ ID:82 and SEQ ID:61;
SEQ ID:14, SEQ ID:83 and SEQ ID:62;
SEQ ID:15, SEQ ID:84 and SEQ ID:63;
SEQ ID:16, SEQ ID:85 and SEQ ID:64;
SEQ ID:17, SEQ ID:86 and SEQ ID:65;
SEQ ID:18, SEQ ID:87 and SEQ ID:66;
SEQ ID:19, SEQ ID:88 and SEQ ID:67;
SEQ ID:20, SEQ ID:89 and SEQ ID:68;
SEQ ID:22, SEQ ID:90 and SEQ ID:70 and
SEQ ID:24, SEQ ID:91 and SEQ ID:72.

8. The multiplex diagnostic kit of claim 7 further comprising an instruction to perform a multiplex PCR using the forward amplification primers and the backward amplification primers using the same amplification profile.

9. The multiplex diagnostic kit of claim 8 further comprising an instruction to perform a primer extension reaction using the extension primers at the same extension temperature.

10. The multiplex diagnostic kit of claim 9 further comprising an instruction to perform the multiplex PCR and the extension reaction in a single tube.

11. The multiplex diagnostic kit of claim 7 further comprising a biochip to which are coupled in a plurality of distinct positions a plurality of distinct capture probes, respectively, and wherein each of the plurality of capture probes hybridizes with a portion of each of the extension primers, respectively.

12. The multiplex diagnostic kit of claim 11 wherein each of the plurality of the distinct capture probes has a unique sequence distinct from a target nucleic acid to which the amplification primers bind.

13. The multiplex diagnostic kit of claim 7 further comprising at least one of a reagent and an enzyme.

14. A synthetic nucleic acid for use as a PCR primer in a multiplex test that has less than forty nucleotides and comprising an HPV recognition sequence selected from the group consisting of SEQ ID NOs: 1 to 3 and 5 to 24, wherein no more than two nucleotides in the HPV recognition sequence are replaced by A, G, C, or T.

## Patentansprüche

1. Multiplex Diagnostik-Kit, das den gleichzeitigen Nachweis einer Vielzahl von HPV-Genotypen erlaubt, ausgewählt aus 6, 11, 16, 18, 26, 31, 33, 35, 39, 42, 43, 44, 45, 51, 52, 53, 56, 58, 59, 66, 68, 69, 73 und 82, aufweisend: Eine Vielzahl von Amplifizierungsprimer-Paaren und eine Vielzahl von Extensions-Primem, wobei jedes aus der Vielzahl der Amplifizierungsprimer-Paare eine solche Sequenz hat, dass
(a) eine Vielzahl von Amplikons, hergestellt aus einer Ziel-Nukleinsäure unter Verwendung der entsprechenden Vielzahl von Amplifizierungsprimer-Paaren, eine mutierte Position in einer Zielnukleinsäure einschließt;
(b) die Vielzahl von Amplikonen unter Verwendung desselben Amplifizierungs-Profils in einer PCR Reaktion hergestellt wird; und wobei jede der Vielzahl von Extensions-Primem eine solche Sequenz aufweist, dass
(c) jeder aus der Vielzahl der Extensions-Primer spezifisch mit jedem aus der entsprechenden Vielzahl der Amplikons bei der gleichen Extensionstemperatur hybridisiert, wobei das 3'-Ende von jedem der Extensions-Primer mit einer komplementären Position der mutierten Position entsprechend korrespondiert; und
(d) selektive Primerextension für jeden aus der Vielzahl der Extensions-Primer bei der gleichen Extensionstemperatur erreicht wird; was zumindest zwei Amplifizierungsprimer-Paare, einschließlich Forward- und Backward-Amplifizierungsprimer, und zumindest zwei korrespondierende Extensions-Primer mit den folgenden Sequenzen:
SEQ ID:1, SEQ ID:25 und SEQ ID:49;
SEQ ID:2, SEQ ID:26 und SEQ ID:50;
SEQ ID:3, SEQ ID:27 und SEQ ID:51;
SEQ ID:4, SEQ ID:28 und SEQ ID:52;
SEQ ID:5, SEQ ID:29 und SEQ ID:53;
SEQ ID:6, SEQ ID:30 und SEQ ID:54;
SEQ ID:7, SEQ ID:31 und SEQ ID:55;
SEQ ID:8, SEQ ID:32 und SEQ ID:56;
SEQ ID:9, SEQ ID:33 und SEQ ID:57;
SEQ ID:10, SEQ ID:34 und SEQ ID:58;
SEQ ID:11,SEQ ID:35 und SEQ ID:59;
SEQ ID:12, SEQ ID:36 und SEQ ID:60;
SEQ ID:13, SEQ ID:37 und SEQ ID:61;
SEQ ID:14, SEQ ID:38 und SEQ ID:62;
SEQ ID:15, SEQ ID:39 und SEQ ID:63;
SEQ ID:16, SEQ ID:40 und SEQ ID:64;
SEQ ID:17, SEQ ID:41 und SEQ ID:65;
SEQ ID:18, SEQ ID:42 und SEQ ID:66;
SEQ ID:19,SEQ ID:43 und SEQ ID:67;
SEQ ID:20, SEQ ID:44 und SEQ ID:68;
SEQ ID:21, SEQ ID:45 und SEQ ID:69;
SEQ ID:22, SEQ ID:46 und SEQ ID:70;
SEQ ID:23, SEQ ID:47 und SEQ ID:71 und
SEQ ID:24, SEQ ID:48 und SEQ ID:72
einschließt,
wobei die Primer mit degenerierten Positionen durch individuelle Sequenzen oder durch eine Mischung der Sequenzen repräsentiert werden können.

2. Multiplex Diagnose-Kit nach Anspruch 1, ferner umfassend einen Biochip, an den in einer Vielzahl unterschiedlicher Positionen eine entsprechende Vielzahl unterschiedlicher Erfassungssonden gekoppelt ist, und wobei jede der Vielzahl von Erfassungssonden mit entsprechenden einem Abschnitt von jeden dem Extensions-Primer hybridisiert.

3. Multiplex Diagnose-Kit nach Anspruch 2, wobei jede aus der Vielzahl der unterschiedlichen Erfassungssonden eine einzigartige Sequenz aufweist, die zu der Zielnukleinsäure unterschiedlich ist.

4. Multiplex Diagnose-Kit nach Anspruch 1, die ferner eine DNA-abhängige DNA-Polymerase aufweist.

5. Multiplex Diagnose-Kit nach Anspruch 1, wobei jeder der Extensions-Primer ferner einen eindeutigen Tag an dem 5'-Ende des Extensions-Primers aufweist, um die Hybridisierung mit einer eindeutigen Erfassungssonde zu ermöglichen.

6. Multiplex Diagnose-Kit nach Anspruch 1, das ferner eine Anleitung aufweist, um die PCR-Reaktion und die Primerextension in einer einzigen Röhre auszuführen.

7. Multiplex Diagnose-Kit zum gleichzeitigen Nachweis einer Vielzahl von HPV-Genotypen, ausgewählt aus 16, 18, 26, 31, 33, 35, 39, 45, 51, 52, 53, 56, 58, 59, 66, 69 und 82, aufweisend zumindest zwei Forward-Amplifizierungsprimer und zumindest zwei Backward-Amplifizierungsprimer und korrespondierende Extensions-Primer mit den folgenden Sequenzen:
SEQ ID:73, SEQ ID:75 und SEQ ID:51;
SEQ ID:4, SEQ ID:76 und SEQ ID:52;
SEQ ID:5, SEQ ID:77 und SEQ ID:53;
SEQ ID:6, SEQ ID:78 und SEQ ID:54;
SEQ ID:74, SEQ ID:79 und SEQ ID:55;
SEQ ID:8, SEQ ID:80 und SEQ ID:56;
SEQ ID:9, SEQ ID:81 und SEQ ID:57;
SEQ ID:13,SEQ ID:82 und SEQ ID:61;
SEQ ID: 14, SEQ ID:83 und SEQ ID:62;
SEQ ID:15, SEQ ID:84 und SEQ ID:63;
SEQ ID:16, SEQ ID:85 und SEQ ID:64;
SEQ ID:17, SEQ ID:86 und SEQ ID:65;
SEQ ID:18, SEQ ID:87 und SEQ ID:66;
SEQ ID:19, SEQ ID:88 und SEQ ID:67;
SEQ ID:20, SEQ ID:89 und SEQ ID:68;
SEQ ID:22, SEQ ID:90 und SEQ ID:70 und
SEQ ID:24, SEQ ID:91 und SEQ ID:72.

8. Multiplex Diagnose-Kit nach Anspruch 7, das ferner eine Anleitung aufweist, um ein Multiplex-PCR unter Verwendung der Forward-Amplifizierungsprimer und der Backward-Amplifizierungsprimer, die dasselbe Amplifizierungsprofil verwenden, auszuführen.

9. Multiplex Diagnose-Kit nach Anspruch 8, das ferner eine Anleitung aufweist, um eine Primerextensionsreaktion unter Verwendung der Extensions-Primer bei derselben Extensionstemperatur auszuführen.

10. Multiplex Diagnose-Kit nach Anspruch 9, das ferner eine Anleitung aufweist, um die Multiplex PCR und die Extraktionsreaktion in einer einzigen Röhre auszuführen.

11. Multiplex Diagnose-Kit nach Anspruch 7, ferner umfassend einen Biochip, auf dem eine Vielzahl unterschiedlicher Erfassungssonden in einer entsprechenden Vielzahl unterschiedlicher Positionen gekoppelt sind, und wobei jede aus der Vielzahl der Erfassungssonden mit entsprechend einem Abschnitt von jedem der Extensions-Primer hybridisiert.

12. Multiplex Diagnose-Kit nach Anspruch 11, wobei jede aus der Vielzahl der unterschiedlichen Erfassungssonden eine einzigartige, von einer Zielnukleinsäure verschiedene Sequenz hat, an die die Amplifizierungsprimer binden.

13. Multiplex Diagnose-Kit nach Anspruch 7, das ferner zumindest ein Reagenz oder ein Enzym aufweist.

14. Synthetische Nukleinsäure zur Verwendung als PCR-Primer in einem Multiplextest, die weniger als vierzig Nukleotide aufweist und eine HPV-Erkennungssequenz aufweist, ausgewählt aus der Gruppe bestehend aus SEQ ID-Nummern: 1 bis 3 und 5 bis 24, wobei nicht mehr als zwei Nukleotide in der HPC-Erkennungssequenz durch A, G, C, oder T ersetzt sind.

## Revendications

1. Kit de diagnostic multiplex qui permet la détection simultanée d'une pluralité de génotypes de HPV choisis parmi 6, 11, 16, 18, 26, 31, 33, 35, 39, 42, 43, 44, 45, 51, 52, 53, 56, 58, 59, 66, 68, 69, 73 et 82 comprenant : une pluralité de paires d'amorces d'amplification et une pluralité d'amorces d'extension, où chacune de la pluralité de paires d'amorces d'amplification a une séquence telle que
(a) une pluralité d'amplicons produits à partir d'un acide nucléique cible en utilisant la pluralité de paires d'amorces d'amplification, respectivement, inclut une position mutée dans un acide nucléique cible ;
(b) la pluralité d'amplicons est produite dans une réaction PCR en utilisant le même profil d'amplification ; et où chacune de la pluralité d'amorces d'extension a une séquence telle que
(c) chacune de la pluralité d'amorces d'extension s'hybride spécifiquement sur chacun de la pluralité d'amplicons à la même température d'extension, respectivement, de telle sorte que l'extrémité 3' de chacune des amorces d'extension correspond à une position complémentaire de la position mutée, respectivement ; et
(d) l'extension d'amorce sélective pour chacune de la pluralité d'amorces d'extension est atteinte à la même température d'extension ;
qui inclut au moins deux paires d'amorces d'amplification incluant des amorces d'amplification sens et antisens et au moins deux amorces d'extension correspondantes ayant les séquences suivantes :
SEQ ID : 1, SEQ ID : 25 et SEQ ID : 49 ;
SEQ ID : 2, SEQ ID : 26 et SEQ ID : 50 ;
SEQ ID : 3, SEQ ID : 27 et SEQ ID : 51 ;
SEQ ID : 4, SEQ ID : 28 et SEQ ID : 52 ;
SEQ ID : 5, SEQ ID : 29 et SEQ ID : 53 ;
SEQ ID : 6, SEQ ID : 30 et SEQ ID : 54 ;
SEQ ID : 7, SEQ ID : 31 et SEQ ID : 55 ;
SEQ ID : 8, SEQ ID : 32 et SEQ ID : 56 ;
SEQ ID : 9, SEQ ID : 33 et SEQ ID : 57 ;
SEQ ID : 10, SEQ ID : 34 et SEQ ID : 58 ;
SEQ ID : 11, SEQ ID : 35 et SEQ ID : 59 ;
SEQ ID : 12, SEQ ID : 36 et SEQ ID : 60 ;
SEQ ID : 13, SEQ ID : 37 et SEQ ID : 61 ;
SEQ ID : 14, SEQ ID : 38 et SEQ ID : 62 ;
SEQ ID : 15, SEQ ID : 39 et SEQ ID : 63 ;
SEQ ID : 16, SEQ ID : 40 et SEQ ID : 64 ;
SEQ ID : 17, SEQ ID : 41 et SEQ ID : 65 ;
SEQ ID : 18, SEQ ID : 42 et SEQ ID : 66 ;
SEQ ID : 19, SEQ ID : 43 et SEQ ID : 67 ;
SEQ ID : 20, SEQ ID : 44 et SEQ ID : 68 ;
SEQ ID : 21, SEQ ID : 45 et SEQ ID : 69 ;
SEQ ID : 22, SEQ ID : 46 et SEQ ID : 70 ;
SEQ ID : 23, SEQ ID : 47 et SEQ ID : 71 et
SEQ ID : 24, SEQ ID : 48 et SEQ ID : 72
moyennant quoi les amorces à des positions dégénérées peuvent être représentées par les séquences individuelles ou par un mélange des séquences.

2. Kit de diagnostic multiplex selon la revendication 1, comprenant en outre une biopuce à laquelle sont couplés dans une pluralité de positions distinctes une pluralité de sondes de capture distinctes, respectivement, et où chacune de la pluralité de sondes de capture s'hybride avec une portion de chacune des amorces d'extension, respectivement.

3. Kit de diagnostic multiplex selon la revendication 2, dans lequel chacune de la pluralité des sondes de capture distinctes a une séquence unique distincte de l'acide nucléique cible.

4. Kit de diagnostic multiplex selon la revendication 1, comprenant en outre une ADN polymérase dépendante de l'ADN.

5. Kit de diagnostic multiplex selon la revendication 1, dans lequel chacune des amorces d'extension comprend en outre une étiquette unique à l'extrémité 5' des amorces d'extension pour permettre l'hybridation avec une sonde de capture unique.

6. Kit de diagnostic multiplex selon la revendication 1, comprenant en outre une instruction pour effectuer la réaction PCR et une extension d'amorce dans un tube unique.

7. Kit de diagnostic multiplex pour la détection simultanée d'une pluralité de génotypes de HPV choisis parmi 16, 18, 26, 31, 33, 35, 39, 45, 51, 52, 53, 56, 58, 59, 66, 69 et 82, comprenant au moins deux amorces d'amplification sens et au moins deux amorces d'amplification antisens et des amorces d'extension correspondantes ayant les séquences suivantes :
SEQ ID : 73, SEQ ID : 75 et SEQ ID : 51 ;
SEQ ID : 4, SEQ ID : 76 et SEQ ID : 52 ;
SEQ ID : 5, SEQ ID : 77 et SEQ ID : 53 ;
SEQ ID : 6, SEQ ID : 78 et SEQ ID : 54 ;
SEQ ID : 74, SEQ ID : 79 et SEQ ID : 55 ;
SEQ ID : 8, SEQ ID : 80 et SEQ ID : 56 ;
SEQ ID : 9, SEQ ID : 81 et SEQ ID : 57 ;
SEQ ID : 13, SEQ ID : 82 et SEQ ID : 61 ;
SEQ ID : 14, SEQ ID : 83 et SEQ ID : 62 ;
SEQ ID : 15, SEQ ID : 84 et SEQ ID : 63 ;
SEQ ID : 16, SEQ ID : 85 et SEQ ID : 64 ;
SEQ ID : 17, SEQ ID : 86 et SEQ ID : 65 ;
SEQ ID : 18, SEQ ID : 87 et SEQ ID : 66 ;
SEQ ID : 19, SEQ ID : 88 et SEQ ID : 67 ;
SEQ ID : 20, SEQ ID : 89 et SEQ ID : 68 ;
SEQ ID : 22, SEQ ID : 90 et SEQ ID : 70 et
SEQ ID : 24, SEQ ID : 91 et SEQ ID : 72.

8. Kit de diagnostic multiplex selon la revendication 7, comprenant en outre une instruction pour effectuer une PCR multiplex à l'aide des amorces d'amplification sens et des amorces d'amplification antisens au moyen du même profil d'amplification.

9. Kit de diagnostic multiplex selon la revendication 8, comprenant en outre une instruction pour effectuer une réaction d'extension d'amorce à l'aide des amorces d'extension à la même température d'extension.

10. Kit de diagnostic multiplex selon la revendication 9, comprenant en outre une instruction pour effectuer la PCR multiplex et la réaction d'extension dans un tube unique.

11. Kit de diagnostic multiplex selon la revendication 7, comprenant en outre une biopuce à laquelle sont couplées dans une pluralité de positions distinctes une pluralité de sondes de capture distinctes, respectivement, et où chacune de la pluralité de sondes de capture s'hybride avec une portion de chacune des amorces d'extension, respectivement.

12. Kit de diagnostic multiplex selon la revendication 11, dans lequel chacune de la pluralité des sondes de capture distinctes a une séquence unique distincte d'un acide nucléique cible auquel les amorces d'amplification se lient.

13. Kit de diagnostic multiplex selon la revendication 7, comprenant en outre au moins l'un parmi un réactif et une enzyme.

14. Acide nucléique synthétique à utiliser comme amorce PCR dans un essai multiplex qui comporte au moins quarante nucléotides et comprenant une séquence de reconnaissance de HPV choisie dans le groupe consistant en les SEQ ID NO : 1 à 3 et 5 à 24, où pas plus de deux nucléotides dans la séquence de reconnaissance de HPV sont remplacés par A, G, C ou T.
